# FASCICULE DE BREVET EUROPEEN

(11) **EP 0 607 129 B1**
(45) Date de publication et mention de la délivrance du brevet: **23.08.1995**
(21) Numéro de dépôt: 91916897.1
(22) Date de dépôt: 10.10.1991
(51) Int. Cl.: C07D 471/04, C07D 498/04, C07D 513/04, C07D 517/04, A61K 31/55

(54) **DERIVES DE METHYLPIPERAZINOAZEPINE, LEUR PREPARATION ET LEUR UTILISATION**
DERIVATE VON METHYLPIPERAZINOAZEPIN, VERFAHREN ZU IHRER HERSTELLUNG UND VERWENDUNG
METHYLPIPERAZINOAZEPINE DERIVATIVES, THEIR PREPARATION AND USE

(43) Date de publication de la demande: 27.07.1994
(73) Titulaire: "THERABEL RESEARCH SA/NV", B-1180 Bruxelles (BE)
(72) Inventeur: LIEGEOIS, Jean-François, Fernand, B-4040 Herstal (BE); DELARGE, Jacques, Elie, B-4140 Dolembreux (BE)
(74) Mandataire: Claeys, Pierre
(86) Numéro de dépôt international: BE9100073
(87) Numéro de publication internationale: WO9307143

(56) Documents cités:
- EP-A- 0 003 016
- EP-A- 0 354 781
- EP-A- 0 413 300
- US-A- 3 872 122
- US-A- 4 163 785
- US-A- 4 337 198
- Acta Cryst., vol. C47, 15 août 1991, pages 1740-1742, L. Dupont et al.: "Structure du (Méthyl-4 pipérazinyl-1)-10 Pyrido[4,3-b][1,4]benzothiazépine"

## Description

La présente invention est relative à de nouveaux dérivés de méthylpipérazinoazépine et à leurs sels non toxiques ainsi qu'à leur utilisation thérapeutique.

Les nouvelles méthylpipérazinoazépines de l'invention sont représentées par la formule générale (I) : 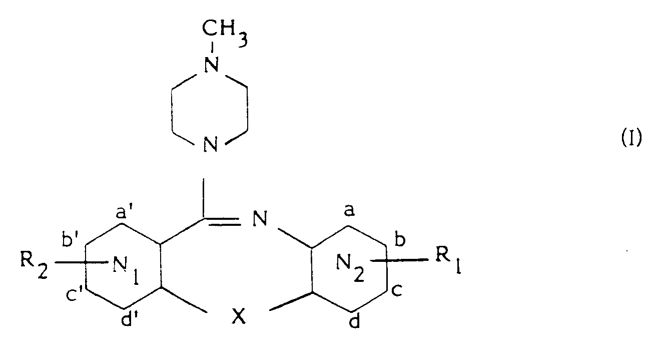
dans laquelle :
- X: représente un atome d'oxygène, de soufre ou de sélénium ou un groupe NH ou NR₃, où R₃ représente un groupe 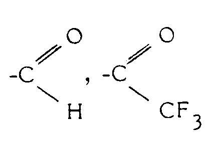 ou alkyle de 1 à 4 atomes de carbone, ramifié ou non;
- R₁: représente un atome d'hydrogène, un atome d'halogène ou un groupe alkyle de 1 à 4 atomes de carbone, ramifié ou non;
- R₂: représente un atome d'hydrogène, un atome d'halogène ou un groupe alkyle de 1 à 4 atomes de carbone, ramifié ou non; et
- N₁: représente un noyau benzénique et N₂ un noyau pyridinique ou inversement, où l'atome d'azote du noyau pyridinique est en position b, b', d ou d', et à la condition que, lorsque R₁ et R₂ représentent de l'hydrogène et X représente du soufre, de l'oxygène ou un groupe NH, N₁ soit pyridinique et N₂ benzénique, l'azote pyridinique ne pouvant pas être en position d', et N₁ et N₂ peuvent en outre être tous deux benzéniques lorsque X représente un atome de sélénium,
et le dérivé 6-(4-méthylpipérazin-1-yl)dipyrido(2,3-b:3',2'-f)1,4-thiazépine, à l'exclusion de la 10-(4-méthylpipérazin-1-yl) pyrido[4,3-b][1,4]benzothiazépine.

Un certain nombre de dérivés de méthylpipérazinoazépine de ce type ont déjà été rapportés dans la littérature. On se référera, à cet égard, en particulier aux articles de Dupont et coll., Acta cryst., C43, 716-718, 1987 pour le dérivé oxygéné, Hoffmann et coll., brevet des Etats-Unis d'Amérique n° 4.163.785, 1979 pour le composé soufré et Chakrabarti et coll., Journal of Medicinal Chemistry, vol. 32, n° 10, 2375-2381, 1989 pour le dérivé azoté. Toutefois, contrairement aux dérivés de la présente invention, aucune méthylpipérazinoazépine de ce type décrite jusqu'à présent n'a présenté d'activité pharmacologique intéressante. On se référera également au document EP-A-0 413 300 décrivant des pyrido[3,4-b][1,4]benzoxazépines, la 6-(4-méthyl-1-pipérazinyl)pyrido[3,4-b][1,4]benzoxazépine et les benzoxazépines de la formule 6 de la page 12 dudit document se rapprochant le plus structurellement des dérivés de l'invention. Toutefois, ces benzoxazépines ont l'atome d'azote du noyau pyridinique en positon c, ce qui les exclut de la formule générale I.

Ainsi qu'on l'a déjà précisé précédemment, les dérivés de méthylpipérazinoazépine de la présente invention sont représentés par la formule générale (I). On entend, dans la formule générale précitée, par groupe alkyle de 1 à 4 atomes de carbone, ramifié ou non, les groupes méthyle, éthyle, n-propyle, isopropyle, n-butyle, isobutyle et tert-butyle. Le terme halogène couvre, quant à lui, le chlore, le fluor, l'iode et le brome.

Des classes préférées de composés suivant la formule (I) sont, notamment, celle dans laquelle R₁ représente un atome d'hydrogène, un atome de chlore, un atome de fluor ou un groupe méthyle et celle dans laquelle R₂ représente un atome d'hydrogène ou un atome de chlore. Une classe particulièrement préférée de ces composés est celle dans laquelle R₁ représente de l'hydrogène, du chlore, du fluor ou du méthyle et, simultanément, R₂ représente de l'hydrogène ou du chlore.

Les dérivés suivant la formule (I) qui peuvent se présenter sous la forme de sels non toxiques, sont en particulier des sels d'acides inorganiques, tels que chlorhydrates, bromhydrates, phosphates, sulfates, ou d' acides organiques, comme les acétates, les citrates, les maléates, les fumarates et les méthanesulfonates.

Des exemples de dérivés suivant l'invention, outre ceux déjà explicitement exemplifiés, sont :
11-(4-méthylpipérazin-1-yl)-5H-pyrido(4,3-b)benzo-1,5-diazépine;
6-(4-méthylpipérazin-1-yl)pyrido(2,3-b)benzo-1,4-thiazépine;
fumarate de 8-chloro-6-(4-méthylpipérazin-1-yl)pyrido(2,3-b)benzo-1,4-thiazépine;
fumarate de 8-chloro-5-(4-méthylpipérazin-1-yl)pyrido(2,3-b)benzo-1,5-oxazépine;
fumarate de 5-(4-méthylpipérazin-1-yl)-8-méthyl pyrido-(2,3-b)benzo-1,5-oxazépine;
fumarate de 6-(4-méthylpipérazin-1-yl)pyrido(2,3-b)benzo-1,4-oxazépine;
fumarate de 8-chloro-6-(4-méthylpipérazin-1-yl)pyrido(2,3-b)benzo-1,4-oxazépine;
6-(4-méthylpipérazin-1-yl)-11H-pyrido(2,3-b)benzo-1,4-diazépine;
8-chloro-6-(4-méthylpipérazin-1-yl)-11H-pyrido(2,3-b)benzo-1,4-diazépine;
6-(4-méthylpipérazin-1-yl)-8-méthyl-11H-pyrido(2,3-b)benzo-1,4-diazépine;
9-chloro-6-(4-méthylpipérazin-1-yl)-11H-pyrido(2,3-b)benzo-1,4-diazépine;
8-fluoro-6-(4-méthylpipérazin-1-yl)-11H-pyrido(2,3-b)benzo-1,4-diazépine;
5-formyl-11-(4-méthylpipérazin-1-yl)-5H-pyrido(4,3-b)benzo-1,5-diazépine;
11-formyl-5-(4-méthylpipérazin-1-yl)-11H-pyrido(2,3-b)benzo-1,5-diazépine;
11-trifluorométhylcarbonyl-5-(4-méthylpipérazin-1-yl)-11H-pyrido(2,3-b)-benzo-1,5-diazépine;
11-formyl-6-(4-méthylpipérazin-1-yl)-11H-pyrido(2,3-b)benzo-1,4-diazépine;
5-(4-méthylpipérazin-1-yl)dibenzo(b,f)1,4-sélénazépine;
6(4-méthylpipérazin-1-yl)-11-méthyl-11H-pyrido(2,3-b)benzo-1,4-diazépine.

Les nouveaux composés suivant l'invention peuvent être préparés selon le procédé général à partir d'azépinones connues ou aisément synthétisées répondant à la formule (II) : 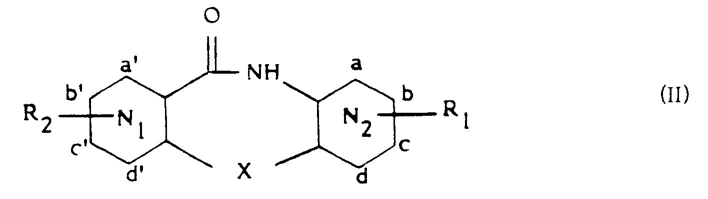
dans laquelle X, R₁ et R₂ sont tels que définis ci-dessus et N₁ et N₂ représentent chacun un noyau benzénique ou pyridinique.

A cet égard, on donne ci-après un certain nombre d'azépinones déjà décrites dans la littérature :
5,11-dihydro-6H-pyrido(2,3-b)benzo-1,4-diazépine-6-one;
6,11-dihydro-5H-pyrido(2,3-b)benzo-1,5-diazépine-5-one;
5,10-dihydro-11H-pyrido(4,3-b)benzo-1,5-diazépine-11-one;
5,11-dihydro-11-méthyl-6H-pyrido(2,3-b)benzo-1,4-diazépine-6-one;
5H,6H-pyrido(2,3-b)benzo-1,5-oxazépine-5-one;
5H,6H-pyrido(2,3-b)benzo-1,4-oxazépine-6-one;
5,11-dihydro-8-méthyl-6H-pyrido(2,3-b)benzo-1,4-diazépine-6-one;
9-chloro-5,11-dihydro-6H-pyrido(2,3-b)benzo-1,4-diazépine-6-one;
10H,11H-dibenzo(b,f)1,4-sélénazépine-11-one.

Cette dernière azépinone, à savoir la 10H,11H-dibenzo(b,f)1,4-sélénazépine-11-one pourrait également être préparée au départ de dibromo-2,2'-benzanilide. Pour ce faire, on introduit dans un ballon par exemple 150 ml de diméthylformamide sec, 0,015 mole de sélénium, 0,050 mole de sodium. On porte le mélange à 100°C, sous agitation pendant 4 heures. Lorsque le tout est dissous, on ajoute 0,025 mole de dibromo-2,2'-benzanilide et on laisse sous agitation pendant 20 heures à 100-110°C. La solution est versée sur un mélange d'acide chlorhydrique et de glace. Le précipité est filtré. Le produit obtenu est traité par de l'alcool bouillant et filtré. Le dérivé est recristallisé dans un mélange de diméthylformamide et d'eau. Point de fusion de 275°C.

On trouvera ci-après la synthèse d'un certain nombre d'azépinones originales non encore décrites dans la littérature.

### Préparation 1

### 8-chloro-5H,6H-pyrido(2,3-b)benzo-1,5-oxazépine-5-one (formule II : X = O, R₁ = Cl en position b, R₂ = H, N₁= pyridine avec N en position d', N₂ = benzène)

Une solution de chlorure de l'acide 2-chloro-pyridine-3-carboxylique (0,1 mole) est versée petit à petit sur une solution de 2-amino-4-chloro-phénol (0,2 mole) dans le tétrahydrofuranne (150 ml). On laisse sous agitation à reflux pendant une heure. On dilue par 1 litre d'eau. Le précipité formé est recueilli, lavé et séché. Le produit brut est utilisé tel quel pour la manipulation suivante. L'oxazépine est préparée en traitant l'amide par la quantité théorique d'éthylate de sodium dans l'alcool absolu. Par évaporation du solvant, le sel sodique est isolé. En chauffant ce dernier à reflux pendant 3 à 4 heures dans le diméthylformamide, on obtient la cyclisation souhaitée. On concentre le mélange par distillation sous vide du DMF et on laisse cristalliser à 0°C. Le produit isolé est lavé par du méthanol froid et recristallisé dans un mélange de DMF/méthanol. Point de fusion de 300°C.

### Préparation 2

### 5H,6H-8-méthyl-pyrido(2,3-b)benzo-1,5-oxazépine-5-one (formule II : X = O, R₁ = CH₃ en position b, R₂ = H, N₁ = pyridine avec N en d', N₂ = benzène)

Cette substance a été préparée suivant le procédé de la préparation 1, mais en utilisant l'acide 2-chloro-pyridine-3-carboxylique et le 2-amino-4-méthylphénol comme matières de départ. Point de fusion : 203°C.

### Préparation 3

### 8-chloro-5H,6H-pyrido(2,3-b)benzo-1,4-oxazépine-6-one (formule II : X = O, R₁ = H, R₂ = Cl en position b', N₁ = benzène, N₂ = pyridine avec N en position d)

On prépare l'ester phénolique de l'acide 5-chlorosalicylique en chauffant à reflux 0,1 mole de phénol et 0,1 mole d'acide 5-chloro-salicylique en présence d'un excès de OPCl₃ pendant 2 heures. Le réactif est chassé sous vide. La masse pâteuse obtenue est reprise progressivement par de l'eau. Le précipité formé est filtré et lavé par de l'eau. Point de fusion de 89°C.

En chauffant à fusion jusqu'à cessation de réaction 0,05 mole de l'ester préparé ci-dessus avec 0,1 mole de 3-amino-2-chloropyridine, on obtient le composé voulu. La masse est reprise par 30 ml d'éthanol et triturée jusqu'à obtention d'un précipité filtrable. Après isolement, le produit est lavé par de l'éthanol et recristallisé dans du dioxanne ou dans un mélange de DMF/méthanol. Point de fusion de 280°C.

### Préparation 4

### 5H,6H-pyrido(2,3-b)benzo-1,4-thiazépine-6-one (formule II : X = S, R₁ = R₂ = H, N₁ = benzène, N₂ = pyridine avec N en position d)

### Procédé 1 :

a) Préparation de l'acide 2-(phénylthio)pyridine-3-carboxylique. On disperse 0,2 mole de thiophénol et 0,2 mole de NaH à 50 % dans 50 cc de propylèneglycol. On ajoute à ce mélange 0,1 mole d'acide 2-chloropyridine-3-carboxylique et on porte à ébullition. Après réaction, on évapore le solvant. On reprend le résidu par de l'eau et on l'amène à pH 7. On extrait l'excès de thiophénol par du chloroforme. La phase aqueuse est purifiée au charbon actif et acidifiée. Le produit précipite sous forme d'une poudre blanche. Point de fusion de 164°C.
b) Le composé précédent est transformé en azide par réaction du chlorure de l'acide préparé ci-dessus. Le chlorure d'acide est versé dans une solution refroidie d'azide sodique en exces. Une fois l'addition terminée, on laisse sous agitation pendant 1/4 d'heure et on dilue par de l'eau. Le produit est isolé et utilisé, après séchage, tel quel pour la réaction suivante. La cyclisation du composé est réalisée par la technique suivante. 1 g d'azide brut est additionné petit à petit à une solution de AlCl₃ dans l'orthodichlorobenzène vers 120°C et sous agitation. La temperature est maintenue pendant 1/2 heure. Le mélange est ensuite repris par du chloroforme et extrait par de l'acide chlorhydrique N/10. La phase acide est réextraite plusieurs fois au CHCl₃. Les phases chloroformiques sont réunies, séchées et concentrées sous vide. Le résidu est repris par de l'acétone et abandonné 2 heures au frigo. Le précipité est recueilli sur filtre. Point de fusion de 206-208°C.

### Procédé 2 :

On chauffe à fusion 0,01 mole de 3-amino-2-chloropyridine jusqu'à cessation de réaction avec 0,01 mole d'acide 2-thiosalicylique. On reprend le résidu, après refroidissement, par 30 ml d'éthanol. On fait bouillir quelques minutes, puis on laisse refroidir. Le précipité est recueilli sur filtre, lavé par de l'éthanol et seché. Point de fusion de 205-208°C.

### Préparation 5

### 5H,6H-pyrido(4,3-b)benzo-1,4-thiazépine-6-one (formule II : X = S, R₁ = R₂ = H, N₁ = benzène, N₂ = pyridine avec N en position b)

On chauffe à reflux pendant quelques heures 0,01 mole d'acide thiosalicylique et 0,01 mole de 3-amino-4-chloropyridine en présence d'orthodichlorobenzène. La réaction terminée, on élimine le solvant. Le résidu est repris par un peu d'eau et amené à pH 5-6. On extrait le composé au chloroforme. Les extraits au CHCl₃ sont évaporés. Le résidu est repris par de l'eau bicarbonatée et agité pendant 1/2 heure. Le compose en suspension est recueilli sur filtre et lavé à l'eau. Point de fusion de 244°C.

### Préparation 6

### 8-chloro-5H,6H-pyrido(2,3-b)benzo-1,4-thiazépine-6-one (formule II : X = S, R₁ = H, R₂ = Cl en position b', N₁ = benzène, N₂ = pyridine avec N en position d)

Le composé est obtenu suivant la méthode décrite dans la Préparation 4, Procédé 1. Point de fusion de 314°C.

### Préparation 7

### 5H,6H-dipyrido(2,3-b:3',2'-f)1,4-thiazépine-5-one (formule II : X = S, R₁ = R₂ = H, N₁ = N₂ = pyridine avec N en position d et d')

A partir de 1,575 g d'acide 2-chloropyridine-3-carboxylique, on prépare le chlorure d'acide par réaction avec le chlorure de thionyle. Après évaporation de l'excès de réactif, le résidu est repris par 20 ml de dioxanne et versé petit à petit sur une solution de 3,12 g de 3-amino-2-mercaptopyridine dans 50 ml de dioxanne sous bonne agitation. On laisse agiter pendant 1/4 d'heure puis on dilue par cinq fois son volume d'eau. Tout passe en solution et le milieu est légèrement acide, le précipité étant éventuellement éliminé. On ajuste le pH vers 7, avec du bicarbonate et on laisse cristalliser. Le produit obtenu est séché à l'étuve ventilée et est recristallisé dans du toluène si nécessaire. Point de fusion de 183°C.

On ajoute 0,01 mole du dérivé amide à une suspension de 0,012 mole de tert-butylate de Na dans 50 ml de DMF. On chauffe à reflux pendant 10-20 heures, on évapore le DMF sous vide et on reprend le résidu par de l'eau (50 ml). Le précipité est filtré, lavé avec de l'eau et séché. Une fois sec, il est repris par de l'éther et trituré. Après filtration et séchage, le produit peut, si nécessaire, être recristallisé dans le DMF. Point de fusion de 305°C.

### Préparation 8

### 8-chloro-5H,6H-pyrido(2,3-b)benzo-1,4-thiazépine-6-one (formule II : X = NH, R₁ = Cl en position b', R₂ = H, N₁ = benzène, N₂ = pyridine avec N en position d)

1) On traite 0,01 mole d'acide 5-chloro-2-nitrobenzoïque par 20 ml de SOCl₂ et quelques gouttes de DMF à reflux pendant une heure. L'excès de SOCl₂ est éliminé, le résidu est repris par 20 ml de dioxanne. Cette solution est additionnée petit à petit à une solution de 0,015 mole de 3-amino-2-chloropyridine, dans 20 ml de dioxanne. Après une heure sous agitation, le mélange est dilué par cinq fois son volume d'eau. Le précipité est filtré, lavé à l'eau froide. Si nécessaire, le produit est recristallisé dans l'isopropanol. Point de fusion de 190°C.
2) On dissout 0,01 mole de dérivé -NO₂ dans 25 ml de HCl concentré. A cette solution, on ajoute petit à petit 11 g de SnCl₂.2H₂O dissous dans 20 ml de HCl concentré. La solution est ensuite portée pendant une heure au bain-marie. Après refroidissement, le précipité est filtré, repris par du NaOH à 10 % et extrait au chloroforme. La phase chloroformique est séchée et concentrée à petit volume en présence de pétroléine 100-140. Le produit cristallisé est ensuite filtré et lavé par de la pétroléine 40-60. Point de fusion de 173°C.
3) On dissout 0,01 mole du dérivé amino préparé selon 2) dans 18 ml de diéthylèneglycolmonométhyléther. On ajoute 0,2 ml de HCl à 5 %. Le mélange est porté à 130°C sous agitation. Après 2-3 heures de chauffage, on obtient une suspension. Dès que la réaction est terminée, on refroidit le mélange et on filtre. Le produit isolé est lavé par du méthanol froid et recristallisé dans du dioxanne si nécessaire. Point de fusion de 296°C.

### Préparation 9

### 5,11-dihydro-8-fluoro-6H-pyrido(2,3-b)benzo-1,4-diazépine-6-one (formule II : X = NH, R₁ = F en position d', R₂ = H, N₁ = benzène, N₂ = pyridine avec N en position d)

Ce composé est préparé suivant le procédé de la Préparation 8, mais à partir d'acide 5-fluoro-2-nitrobenzoïque et de 3-amino-2-chloropyridine. Point de fusion de 270°C.

Le procédé général d'obtention des méthylpypérazinoazépines de formule (I) de l'invention au départ des azépinones de formule (II) peut être exécuté suivant trois modes de réalisation, dont les schémas sont donnés et commentés ci-après. 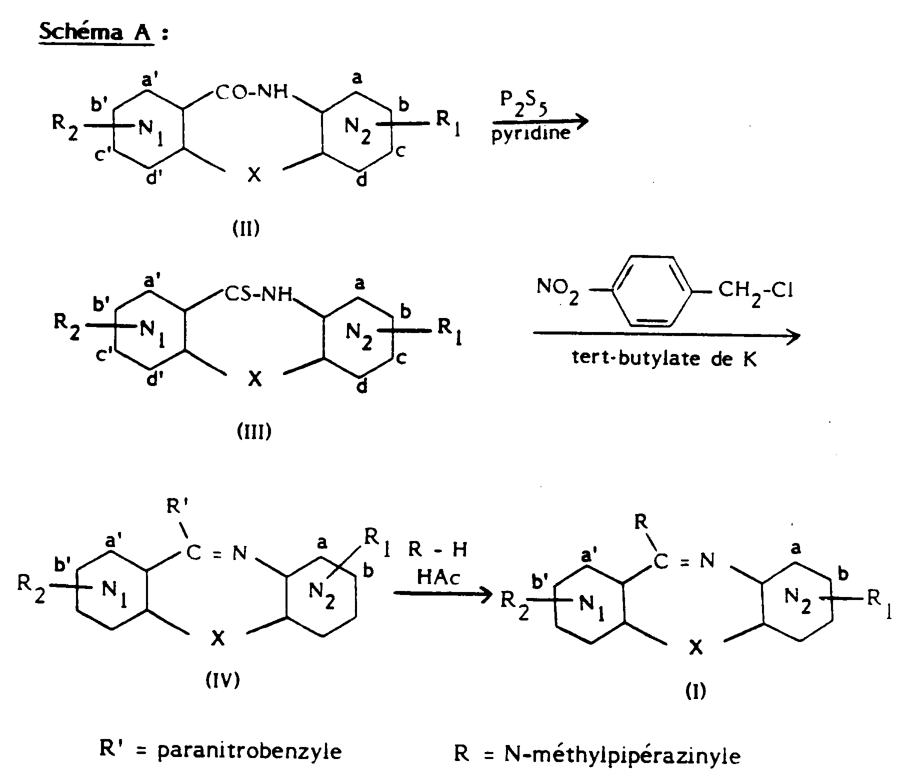
A partir de l'azépinone (II), on synthétise la thione (III) correspondante par action de P₂S₅ dans la pyridine. Le produit brut est isolé, utilisé tel quel et additionné de tert-butylate de potassium et de chlorure de paranitrobenzyle pour la préparation de thioéther (IV). Le composé de formule (I) est obtenu après réaction du thioéther en présence de N-méthylpypérazine et d'acide acétique glacial. (Voir, à cet égard, Hunzicker et coll., Helv. Chim. Acta, 50, 1588, 1967). 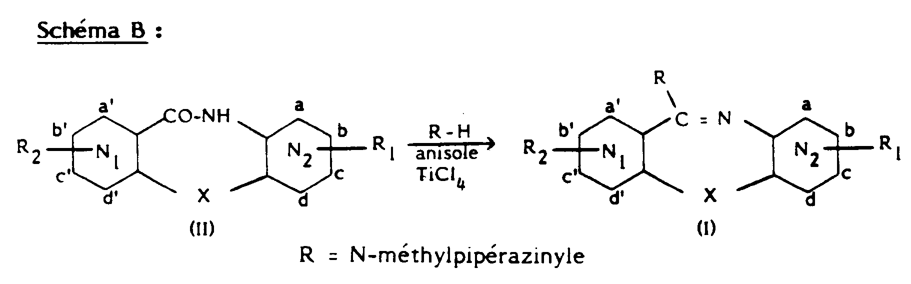
On mélange l'azépinone (II) à de la N-méthylpipérazine en présence d'une solution de TiCl₄ dans l'anisole. Ce mélange permet d'obtenir, sous agitation et chauffage, le composé (I) de l'invention. (Voir, à cet égard, Chakrabarti J. K. et coll., J. Med. Chem. 23, 878, 1980 et Press J. et coll., J. Med. Chem. 22, 725, 1979). 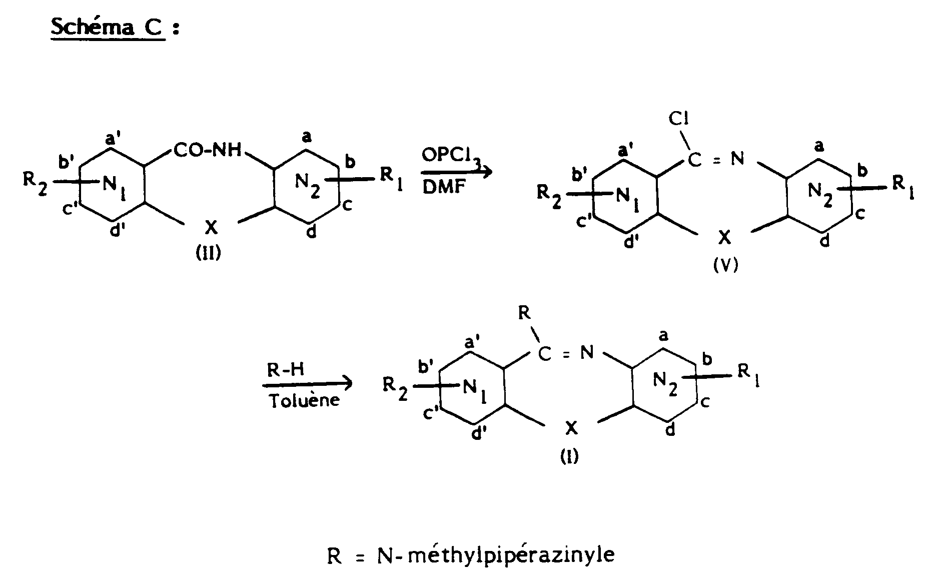
On part de l'azépinone (II) que l'on traite par du OPCl₃ et du DMF pour passer à l'iminochlorure (V) correspondant, que l'on fait réagir sans isolement et condensation subséquente avec la N-méthylpipérazine dans le toluène, pour obtenir le composé (I). (Voir, à cet égard, Hunzicker F. et coll., Helv. Chim. Acta 49 (5), 1933, 1966).

Il est bien entendu également possible de synthetiser les composés de l'invention sans l'utilisation d'azépinones (II) comme matières de départ ou intermédiaires, comme on le verra ci-après dans le cadre de la synthèse de la méthylpipérazinoazépine de l'Exemple 21, au départ du produit de réaction d'une orthohalogéno nitropyridine de formule (V) : 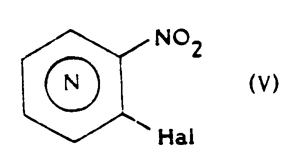
dans laquelle Hal représente un atome d'halogène, tel que Cl, F, I ou Br, et d'un acide benzène carboxylique de formule (VI) : 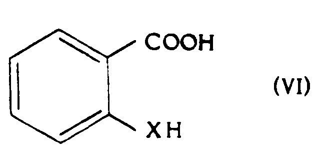
dans laquelle X est tel que défini précédemment.

Les sels des méthylpipérazinoazépines de la formule (I) peuvent être formés par des méthodes bien connues en pratique. D'une façon générale, ces sels peuvent être formés par la reaction en quantité équimoléculaire de la méthylpipérazinoazépine avec un acide dans un solvant adéquat, comme par exemple un alcool, puis éventuellement par précipitation du sel par addition d'un autre solvant miscible au premier et dans lequel le sel est insoluble, par exemple l'éther, ou encore par neutralisation d'une solution éthérée de l' acide ou de la base par la base ou l'acide. Les acides utilisés sont soit des acides organiques soit des acides inorganiques. Comme acide inorganique on utilise de préférence l'acide chlorhydrique, l'acide bromhydrique, l'acide sulfurique, l'acide phosphorique, l'acide perchlorique, etc. Les acides organiques sont soit des acides carboxyliques soit des acides sulfoniques, comme les acides acétique, citrique, maléique, fumarique, propionique, glycolique, lactique, ascorbique, pamoïque, succinique, tartrique, phénylacétique, benzoïque, p-aminobenzoïque, anthranilique, p-hydroxybenzoïque, salicylique, methanesulfonique, éthanedisulfonique, glucuronique, etc.

On donne ci-après des exemples détaillés de préparations de quelques dérivés de méthylpipérazinoazépine suivant l'invention. La préparation des dérivés des Exemples 4 et 18, respectivement connu et exclu du cadre de l'invention en tant que tel, mais non comme agents thérapeutiques, est donnée à titre d'information.

### Exemple 1

### 11-(4-méthylpipérazin-1-yl)-5H-pyrido(4,3-b)benzo-1,5-diazépine (formule I : X = NH, R₁ = R₂ = H, N₁ = pyridine avec N en position b', N₂ = benzène)

### Procédé A :

1) On traite 0,01 mole de 5,10-dihydro-11H-pyrido(4,3-b)benzo-1,5-diazépine-11-one par un excès de P₂S₅ dans de la pyridine chauffée a reflux. Après quelques heures de chauffage, l'excès de solvant et de réactif est éliminé. Le résidu est repris prudemment par de la glace. Le précipité formé est isolé et utilisé tel quel pour la manipulation suivante.
2) 5,2 g du thiolactame sont ajoutés à une suspension de tert-butylate de potassium dans 80 ml de dioxanne (préparé de la façon suivante : 1,64 g de potassium sont dissous dans 40 ml de tert-butanol; après cessation du dégagement gazeux, le solvant est élimine et le résidu est repris par 80 ml de dioxanne). Le mélange est porté au reflux pendant une heure. On ajoute ensuite 4,1 g de chlorure de paranitrobenzyle. On porte à reflux pendant 4 heures. Le solvant est éliminé. Le résidu est repris par du chloroforme et lavé par une lessive alcaline. La phase chloroformique est séchée et évaporée a sec. Le résidu est recristallisé dans un mélange de pétroléine/acétone. Point de fusion de 127°C.
3) 4,3 g du thioéther préparé en 2) sont chauffés à reflux pendant 24 heures en présence de N-méthylpipérazine (10 ml) et de 0,1 ml d'acide acétique glacial. Après évaporation a sec, on reprend le résidu par de l'acide acétique dilué. On filtre l'éventuel précipité. On décolore le filtrat par traitement au charbon actif. La base est précipitée par l'addition d'ammoniaque concentré, filtrée, lavée à l'eau et séchée. Le produit peut être recristallisé dans un mélange de dichlorométhane/hexane. Point de fusion de 216°C.

### Procédé B :

On mélange 0,01 mole de 5,10-dihydro-11H-pyrido-(4,3-b)benzo-1,5-diazépine-11-one à 10 ml de N-méthylpipérazine. Sous agitation, on ajoute prudemment à la suspension de diazépinone une solution de 1,2 ml de TiCl₄ dans 5 ml d'anizole. La réaction est maintenue sous agitation pendant 2-3 heures à 120°C. Le mélange est refroidi et repris par de l'eau glacée. Le pH de la solution doit être alcalin. Le produit est alors extrait par du dichlorométhane. Après évaporation du solvant, on purifie le résidu sur une colonne de kieselgel (phase éluante : acétone/pétroléine 40-60 : 9/1). Après élimination de l'éluant, on recristallise le produit dans l'hexane. Point de fusion de 216°C.

### Exemple 2

### 6-(4-méthylpipérazin-1-yl)pyrido(2,3-b)benzo-1,4-thiazépine (formule I : X = S, R₁ = R₂ = H, N₁ = benzène, N₂ = pyridine avec N en position d)

3 g de 5H,6H-pyrido(2,3-b)benzo-1,4-thiazépine-6-one sont traités par un excès d'oxychlorure de phosphore et 5 gouttes de N,N-diméthylaniline, a reflux pendant 20 heures. La solution est reprise par du toluène anhydre et évaporée à sec. On reprend le résidu par 50 ml de toluène anhydre et on additionne un excès de N-méthylpipérazine. Ce mélange est alors porté à reflux pendant 2-4 heures. Une fois la réaction terminée, on élimine le solvant. La masse colorée est reprise par du chloroforme et lavée deux fois par de l'eau. La phase chloroformique est décolorée par du charbon et séchée. Après concentration en un petit volume, le mélange est passe sur colonne de silice (Woelm act III). La phase éluante est l'acétone. Une fois séparées, les différentes phases contenant le produit cité en rubrique sont évaporées à sec. Le résidu est recristallisé dans la pétroléine 100-140. Point de fusion de 134°C.

### Exemple 3

### Fumarate de 8-chloro-6-(4-méthylpipérazin-1-yl)pyrido(2,3-b)benzo-1,4-thiazépine (formule I : X = S, R₁ = H, R₂ = Cl en b', N₁ = benzène, N₂ = pyridine avec N en position d )

En partant de la 5H,6H-pyrido(2,3-b)benzo-1,4-oxazépine-6-one, on prépare la méthylpipérazinoazépine base citée en rubrique suivant le procédé de l'Exemple 2. Après évaporation des fractions chloroformiques, on dissout le résidu dans un minimum d'alcool bouillant. On ajoute la quantité équimoléculaire d'acide fumarique préalablement dissous dans de l'alcool chaud. La solution est refroidie. Si le produit ne cristallise pas par abandon, on additionne de l'éther jusqu'à cristallisation. Après repos, le précipité blanc obtenu est isolé et lavé à l'éther. Point de fusion de 198°C.

### Exemple 4

### Maléate de 5-(4-méthylpipérazin-1-yl)pyrido(2,3-b)benzo-1,5-oxazépine (formule I : X = O, R₁ = R₂ = H, N₁ = pyridine avec N en position d', N₂ = benzène)

Produit préparé à partir de la 5H,6H-pyrido(2,3-b)-benzo-1,5-oxazépine-5-one et en utilisant le procédé de l'Exemple 2. Les fractions chloroformiques sont ensuite évaporées a sec. Le résidu est repris par de la méthyléthylcétone. On ajoute une quantité équimoléculaire d'acide maléique dissous dans la méthyléthylcétone puis de l'éther anhydre. Le produit cristallise. Il est isolé et lavé à l'éther. Point de fusion de 206°C.

### Exemple 5

### Fumarate de 8-chloro-5-(4-méthylpipérazin-1-yl)pyrido(2,3-b)benzo-1,5-oxazépine (formule I : X = O, R₁ = Cl en position b, R₂ = H, N₁ = pyridine avec N en position d', N₂ = benzène)

A partir de l'oxazépine décrite à la Préparation 1 et en utilisant le procédé de l'Exemple 2, on isole le composé sous forme de fumarate de la même manière que dans l'Exemple 3. Point de fusion de 260°C.

### Exemple 6

### Fumarate de 5-(4-méthylpipérazin-1-yl)-8-méthylpyrido(2,3-b)benzo-1,5-oxazépine (formule I : X = O, R₁ = CH₃ en b, R₂ = H, N₁ = pyridine avec N en position d', N₂ = benzène)

A partir de l'oxazépine décrite à la Préparation 2 et en utilisant le procédé de l'Exemple 2, on isole le composé sous forme de fumarate de la même manière que dans l'Exemple 3. Point de fusion de 235°C.

### Exemple 7

### Fumarate de 6-(4-méthylpipérazin-1-yl)pyrido(2,3-b)benzo-1,4-oxazépine (formule I : X = O, R₁ = R₂ = H, N₁ = benzène, N₂ = pyridine avec N en position d)

A partir de 5H,6H-pyrido(2,3-b)benzo-1,4-oxazépine-6-one et en utilisant le procédé de l'Exemple 2, on isole le composé sous forme de fumarate. Point de fusion de 183°C.

### Exemple 8

### Fumarate de 8-chloro-6-(4-méthylpipérazin-1-yl)pyrido(2,3-b)benzo-1,4-oxazépine (formule I : X = O, R₁ = H, R₂ = Cl en position b', N₁ = benzène, N₂ = pyridine avec N en position d)

A partir de la 8-chloro-5H,6H-pyrido(2,3-b)benzo-1,4-oxazépine-6-one de la Préparation 3, on prépare le composé suivant le procédé décrit dans l'Exemple 2. Le composé est isolé sous la forme de fumarate de la même manière que dans l'Exemple 3. Point de fusion de 250°C.

### Exemple 9

### 6-(4-méthylpipérazin-1-yl)-11H-pyrido(2,3-b)benzo-1,4-diazépine (formule I : X = NH, R₁ = R₂ = H, N₁ = benzène, N₂ = pyridine avec N en position d)

A partir de la 5,11-dihydro-6H-pirido(2,3-b)benzo-1,4-diazépine-6-one, on réalise la condensation suivant le procédé B de l'Exemple 1. Point de fusion de 141°C.

### Exemple 10

### 8-chloro-6-(4-méthylpipérazin-1-yl)-11H-pyrido(2,3-b)benzo-1,4-diazépine (formule I : X = NH, R₁ = Cl en b', R₂ = H, N₁ = benzène, N₂ = pyridine avec N en d)

A partir de la thiazépinone de la Préparation 8, on obtient le produit cité en rubrique suivant le mode opératoire décrit dans l'Exemple 1, Procédé B. Point de fusion de 180°C.

### Exemple 11

### 6-(4-méthylpipérazin-1-yl)-8-méthyl-11H-pyrido(2,3-b)benzo-1,4-diazépine (formule I : X = NH, R₁ = CH₃ en b', R₂ = H, N₁ = benzène, N₂ = pyridine avec N en d)

A partir de la 5,11-dihydro-8-méthyl-6H-pyrido(2,3-b)-benzo-1,4-thiazépine-6-one, on prépare le composé cité en rubrique suivant le mode opératoire de l'Exemple 1, procédé B. Point de fusion de 157°C.

### Exemple 12

### 9-chloro-6-(4-méthylpipérazin-1-yl)-11H-pyrido(2,3-b)benzo-1,4-diazépine (formule I : X = NH, R₁ = Cl en c', R₂ = H, N₁ = benzène, N₂ = pyridine avec N en d)

A partir de la 9-chloro-5,11-dihydro-6H-pyrido(2,3-b)-benzo-1,4-diazépine-6-one, on prépare le produit cité en rubrique suivant le mode opératoire de l'Exemple 1, Procédé B. Point de fusion de 186°C.

### Exemple 13

### 8-fluoro-6-(4-méthylpipérazin-1-yl)-11H-pyrido(2,3-b)benzo-1,4-diazépine (formule I : X = NH, R₁ = F en b', R₂ = H, N₁ = benzène, N₂ = pyridine avec N en d)

A partir de la diazépinone de la Préparation 9, on prépare le composé cité en rubrique suivant le mode opératoire de l'Exemple 1, Procédé B. Point de fusion de 188°C.

### Exemple 14

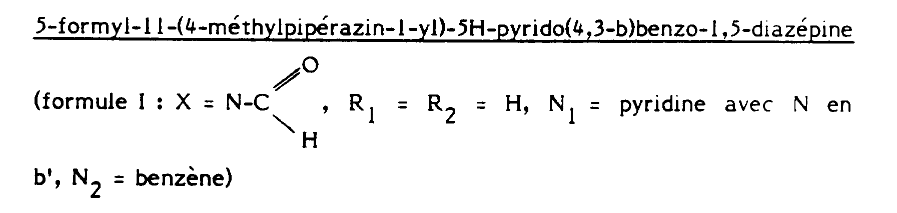

On part du composé de l'Exemple 1. On refroidit 15 cc d'anhydride acétique et on y ajoute petit à petit sous agitation 7 cc d'acide formique à 99 %. On porte le mélange pendant 15 minutes à 50°C puis on le refroidit dans un bain de glace. On ajoute 0,02 mole du composé de l'Exemple 1 et on laisse sous agitation pendant une nuit. On reprend le mélange par de la glace, on alcalinise et on extrait par du dichlorométhane. On sèche et on concentre sous vide, on recristallise dans l'hexane. Point de fusion de 196°C.

### Exemple 15

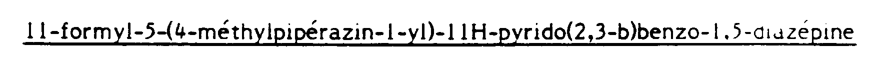

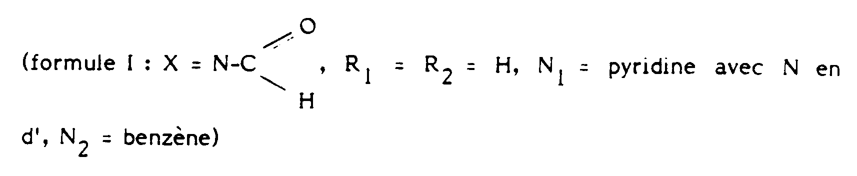

On part du composé décrit dans l'article de Chakrabarti et coll., J. Med. Chem., Vol. 32, n° 10, 2375-2381, 1989 et on réalise la formylation suivant le procédé de l'Exemple 14. Point de fusion de 194°C.

### Exemple 16

### 11-trifluorométhylcarbonyl-5-(4-méthylpipérazin-1-yl)-11H-pyrido(2,3-b)benzo-1,5-diazépine (formule I : X = N - CO - CF₃, R₁ = R₂ = H, N₁ = pyridine avec N en d', N₂ = benzène)

On part du composé décrit dans l'article précité de l'Exemple 15. On mélange 0,02 mole de ce composé avec 15 ml d'anhydride trifluoroacétique et quelques gouttes de N,N-diméthylaniline sous bon refroidissement, puis on porte à reflux pendant 5 minutes et on laisse sous agitation pendant une nuit. On verse sur de la glace, on alcalinise, on extrait au chloroforme. On purifie sur colonne de silice et on concentre. On recristallise dans l'hexane. Point de fusion de 183°C.

### Exemple 17

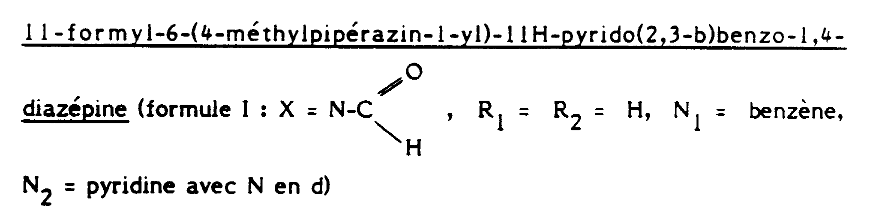

On part du composé de l'Exemple 9 et on réalise la formylation selon le mode opératoire de l'Exemple 14. Point de fusion de 202°C.

### Exemple 18

### 10-(4-méthylpipérazin-1-yl)pyrido(4,3-b)benzo-1,4-thiazépine (formule I : X = S, R₁ = R₂ = H, N₁ = benzène, N₂ = pyridine avec N en b)

A partir de la thiazépinone de la Préparation 5, on obtient le composé cité en rubrique en utilisant le procédé de l'Exemple 2. Point de fusion de 143-144°C.

### Exemple 19

### 5-(4-méthylpipérazin-1-yl)dibenzo(b,f)1,4-sélénazépine (formule I : X = Se, R₁ = R₂ = H, N₁ = N₂ = benzène)

A partir de la 10H,11H-dibenzo(b,f)1,4-sélénazépine-11-one, on obtient le composé cité en rubrique en utilisant le procédé de l'Exemple 2. Point de fusion de 107°C.

### Exemple 20

### 6-(4-méthylpipérazin-1-yl)dipyrido(2,3-b:3',2'-f)1,4-thiazépine (formule I : X = S, R₁ = R₂ = H, N₁ = N₂ = pyridine avec N en d et d')

Produit préparé à partir du composé de la Préparation 7, en utilisant le procédé de l'Exemple 2. Point de fusion de 170°C.

### Exemple 21

### 6-(4-méthylpipérazin-1-yl)-11-méthyl-11H-pyrido(2,3-b)benzo-1,4-diazépine (formule I : X = N-CH₃, R₁ = R₂ = H, N₁ = benzène, N₂ = pyridine avec N en position d)

0,02 mole de 2-chloro-3-nitropyridine, 0,01 mole d'acide N-méthyl anthranylique, 5 g de K₂CO₃ anhydre et 50 ml d'isopropanol sec, sont chauffés à reflux pendant 24 heures. Après évaporation du solvant, on reprend le résidu par de l'eau et on le porte à ébullition en présence de charbon actif. La solution, après refroidissement, est acidifiée aux environs de pH 3. Le produit précipite sous forme de poudre jaune, et il est filtré, lavé à l'eau et séché. Point de fusion de 174°C.

On met en suspension dans l'éther anhydre 0,005 mole de l'acide obtenu. Le composé est estérifié par le diazométhane, que l'on prépare à partir de 4 g de nitrosométhylurée par décomposition de celle-ci dans la soude en présence d'éther. La solution éthérée de diazométhane est versée petit à petit sur la solution éthérée de l'acide nitré. Une fois la réaction terminée, on évapore le solvant. On reprend le résidu par une solution de NaHCO₃ et on extrait deux fois par du CHCl₃. La solution chloroformique est séchée et concentrée en présence de pétroléine 100-140. Le produit cristallisé est ensuite filtré et lavé par de la pétroléine 40-60. Point de fusion de 71°C.

0,01 mole de dérivé nitroester (solubilisé dans 150 ml d'éthanol) est réduite catalytiquement par du charbon palladié (Pd/C 10 %; 1 g) dans un hydrogénateur basse pression. Après 2 heures, la réaction est terminée et le solvant est évaporé. Le résidu (aminoester) est repris par 50 ml d'anisole et transvasé dans un ballon. On ajoute 20 ml de N-méthylpipérazine. On porte le mélange à 120°C sous agitation. Une solution de 5 ml de TiCl₄ dans 10 ml d'anisole est versée prudemment sur le mélange. Le milieu réactionnel est maintenu pendant 12 heures à reflux. Le mélange est ensuite refroidi et repris par 10 ml d'isopropanol, 10 ml d'ammoniaque concentrée et 2 g de silice. Le mélange est filtré et le précipité recueilli est lavé soigneusement par du chloroforme. Le filtrat est lavé une fois par de l'eau, puis est extrait par du HCl 2N. Cette solution est décolorée par du charbon actif et alcalinisée par de l'ammoniaque. On extrait le précipité formé par du chloroforme. Les fractions chloroformiques sont rassemblées et concentrées en un petit volume. Le résidu obtenu est purifié sur une colonne de Kieselgel avec un mélange 9/1 d'acétone et de pétroléine 40-60 comme phase éluante. Une fois isolé, on recristallise le produit dans un mélange de dichlorométhane et d'hexane. Point de fusion de 146°C.

Les composés de l'invention et le composé de l'Exemple 18 ont été étudiés dans des tests pharmacologiques susceptibles de révéler une activité au niveau du système nerveux central ou périphérique. Les résultats suivants obtenus sur les substances des Exemples 1, 10, 11, 17, 18 et 19, confirment ces hypothèses et, par conséquent, l'activité antidépressive, antipsychotique, anxiolytique, neuroleptique ou sédative des composés en question.

### In vitro : Tests d'affinité sur récepteurs dopaminergiques D2 et muscarinergiques

| Code | Inhibition, % (binding de ³H-spipérone) | Inhibition, % (binding de ³H QNB) |
|---|---|---|
| Clozapine | 57,3 | 80,35 |
| Clothiapine | 94,6 | 38,05 |
| Halopéridol | 100 | 0 |
| Exemple 1 | 56,2 | 4,48 |
| Exemple 10 | 31,5 | 62,08 |
| Exemple 11 | 24,8 | 67,3 |
| Exemple 17 | 22,8 | 43,38 |
| Exemple 18 | 50,2 | 57,61 |
| Exemple 19 | 26,7 | 78,31 |

### Récepteurs dopaminergiques D2 :

Caractérisation par essai de déplacement de la (³H) spipérone.

### Préparation des membranes :

Le cerveau de rats mâles Wistar (200-250 g) est prélevé. Le striatum est disséqué rapidement et homogénéisé dans 20 ml de tampon glacé (50nM Tris/HCl, pH = 7,4, 25°C) en utilisant un homogénéiseur Teflon. L'homogénat est centrifugé à 4°C à 10000 g pendant 10 minutes, le culot est lavé deux fois par du tampon glacé et recentrifugé. Le culot final est mis en suspension dans du tampon glacé (50 nM/HCl, ph = 7,4) contenant 5 nM MgSO₄, 0,5 nM EDTA. Après réhomogénéisation, la suspension est étalonnée à 1 mg de protéine par ml. La quantité de protéine est déterminée par la méthode de Lowry et coll. [J. Biol. Chem. 193, 265-275 (1951)] en utilisant la sérumalbumine bovine comme standard.

### Essai de binding :

Une méthode inspirée de Tecott et coll. [Biol. Psychiatry 21, 1114-1122 (1986)] est utilisée. Le binding de la (³H)-spipérone est évalué sur le mélange suivant : 200 »l de préparation membranaire (0,1-0,2 mg de protéine), 100 »l (10⁻⁶ M) de substance test et 600 »l de tampon pour un volume final de 1 ml. Des tubes contrôles, pris comme témoin du binding non spécifique, contiennent aussi 1 »M de (+) butaclamol. Les essais (en trois exemplaires) sont ensuite incubés avec 0,5 nM de (³H)spipérone à 25°C pendant 60 minutes. La manipulation est arrêtée en filtrant rapidement sous vide la suspension au travers d'un filtre en fibres de verre GF/C. Le filtre est rincé trois fois avec 5 ml de tampon glacé. La radioactivité est mesurée avec un spectromètre à scintillation liquide (LKB Rack Beta 1219). Les résultats sont donnés par la différence de pourcentage entre le contrôle (1 »M (+)butaclamol) et la substance testée.

### Caractérisation des récepteurs muscariniques

### Liaisons aux récepteurs :

La méthode est inspirée de la technique décrite par Yamamura et Snyder [Proc. Natl. Sci. USA 71, 1725 (1974)]. Apres un prélèvement rapide, le cerveau de rat CFY (130-180 g) est disséqué pour éliminer le cervelet. Les autres tissus sont homogénéisés avec un "Potter" dans une solution de saccharose 0,32 M, le tampon Tris/HCl 50 mM pH 7,5. La concentration en protéine est déterminée selon la méthode de Peterson [Anal. Biochem. 83, 346 (1977)].

L'essai de binding est réalisé en trois exemplaires a 25°C. Le milieu d'incubation (1 ml) est constitué de NaCl 60 mM, de tampon Tris/HCl pH 7,5 et de 1,5 nM de (³H) QNB (New England Nuclear, act. spécifique : 1,18 TBq/mole). La réaction est maintenue pendant 60 minutes avec un homogénat contenant 250-400 »g de protéine. Le binding non spécifique est réalisé par de l'atropine 10⁻⁵M. L'arrêt de la manipulation est réalisé par filtration rapide sur filtre de verre Whatman GF/C. Le ligand libre est ainsi éliminé. Chaque échantillon est lavé par 2 x 10 ml de tampon froid. Les filtres sont séchés, mis dans une solution toluénique pour scintillation et placés dans un compteur à scintillation (Packard Tricarb liquid scintillation counter) pendant un jour. La liaison au récepteur est directement proportionnelle à la concentration en protéine jusqu'à 700 »g de protéine par ml.

### In vivo : Test d'antagonisme a l'apomorphine

A 20 mg/kg, la plupart des composés diminuent la locomotion chez les animaux et possèdent un effet sédatif. Dans la majorité des cas, les composés présentent une activité dont l'intensité est comprise entre celle de la clozapine (20 mg/g) (+) et celle de l'halopéridol (0,63 mg/kg (+++++). Les résultats suivants sont fournis à titre d'exemple.

| | |
|---|---|
| Exemple 1 | ++ |
| Exemple 5 | + |
| Exemple 7 | + |
| Exemple 8 | +++ |
| Exemple 10 | +++ |
| Exemple 11 | +++ |
| Exemple 12 | + |
| Exemple 13 | + |
| Exemple 19 | ++ |

### Test de catalepsie

Voir notamment GRAY W.D., OSTERBERG A.C., RAUH C.E., Arch. Int. Pharmacodyn. 134, 198, 1961 et COSTALL B., OLLEY J.E., Neuropharmacology 10, 297, 1971.

Ce test est représentatif des effets extrapyramidaux présents dans de nombreux neuroleptiques (effet secondaire non souhaitable). Les composés de l'invention provoquent très peu de catalepsie et paraissent donc dénués de cet effet secondaire non souhaitable. Quelques produits ont toutefois présenté une légère catalepsie :
- Exemples 1 et 3 : catalepsie pour des doses supérieures à 40 mg/kg
- Exemple 4 : léger effet cataleptique à partir de 20 mg/kg
- Clozapine : faible effet cataleptique à partir de 20 mg/kg.

La présente invention a également pour objet des compositions pharmaceutiques qui contiennent comme constituants actifs un ou plusieurs composés de formule (I), le maléate de 5-(4-méthylpipérazin-1-yl)pyrido-(2,3-b)benzo-1,5-oxazépine et les composés 6-(4-méthylpipérazin-1-yl)dipyrido(2,3-b:3',2'-f)1,4-thiazépine et 10-(4-méthylpipérazin-1-yl) pyrido[4,3-b][1,4]benzothiazépine, ou encore un ou plusieurs sels pharmaceutiquement acceptables de ces composés, seuls ou avec d'autres substances actives d'effets similaires ou différents, en mélange avec un excipient pharmaceutique appropriés.

Ces compositions parmaceutiques peuvent être solides comme des comprimes nus ou enrobes, a une ou plusieurs couches, des cachets, des gélules, des poudres dispersables ou solubles, des suppositoires, ou liquides, comme des solutions, des colyres, des suspensions, des émulsions, des sirops, des préparations destinées à l'administration parentérale, par exemple sous forme d'aérosol.

Les compositions solides pour l'usage oral peuvent être préparées en mélangeant une ou plusieurs substances conformes à l'invention par exemple à du sucre de lait, du sucre en poudre, de l'amidon, du talc, à des produits destinés à retarder ou à en prolonger les effets, par exemple l'acétophtalate de cellulose, les stéarates de glycéryle, les résines échangeuses d'ions.

Les suppositoires peuvent être préparés en incorporant une ou plusieurs substances conformes à l'invention à du beurre de cacao par exemple, ou à toute autre substance appropriée, comme les mono-, di- et triglycérides d'acide gras saturés.

Les compositions liquides peuvent être préparées par exemple par dissolution, mise en suspension ou émulsion, au moment de la préparation ou directement avant l'administration, d'une ou plusieurs substances conformes à l'invention et en outre de tout autre produit dont la présence est jugée souhaitable ou nécessaire, comme par exemple des agents conservateurs, tels que les p-hydroxybenzoates de méthyle et de propyle, des épaississants et des émulsionnants comme les dérivés de cellulose et les esters de polyoxyéthylène sorbitane, des édulcorants et aromatisants comme le sucre, la saccharine, le sorbitol, les essences naturelles ou synthétiques, des isotonisants comme le chlorure sodique, ou des tampons comme les phosphates sodiques, dans de l'eau distillée, dans d'autres liquides hydroxylés acceptables, tels qu'éthanol, glycérine, certains glycols, dans des mélanges de ces solvants ou dans des huiles pharmaceutiquement acceptables.

## Revendications

1. Dérivé de méthylpipérazinoazépine répondant à la formule générale (I) : 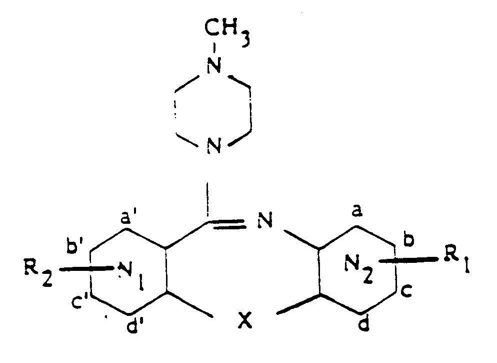 dans laquelle :
X représente un atome d'oxygène, de soufre ou de sélénium ou un groupe NH ou NR₃, où R₃ représente un groupe 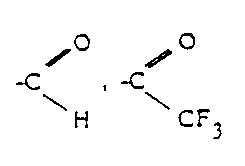 ou alkyle de 1 à 4 atomes de carbone, ramifié ou non;
R₁ représente un atome d'hydrogène, un atome d'halogène ou un groupe alkyle de 1 à 4 atomes de carbone, ramifié ou non;
R₂ représente un atome d'hydrogène, un atome d'halogène ou un groupe alkyle de 1 à 4 atomes de carbone, ramifié ou non; et
N₁ représente un noyau benzénique et N₂ un noyau pyridinique ou inversement, où l'atome d'azote du noyau pyridinique est en position b, b', d ou d', et à la condition que, lorsque R₁ et R₂ représentent de l'hydrogène et X représente du soufre, de l'oxygène ou un groupe NH, N₁ soit pyridinique et N₂ benzénique, l'azote pyridinique ne pouvant pas être en position d', et N₁ et N₂ peuvent en outre être tous deux benzéniques lorsque X représente un atome de sélénium, et
le dérivé 6-(4-méthylpipérazin-1-yl)dipyrido(2,3-b:3',2'-f)1,4-thiazépine, à l'exclusion de la 10-(4-méthylpipérazin-1-yl) pyrido[4,3-b][1,4]benzothiazépine, ou un sel pharmaceutiquement acceptable de ces dérivés.

2. Dérivé suivant la revendication 1, caractérisé en ce que R₁ représente un atome d'hydrogène, un atome de chlore, un atome de fluor ou un groupe méthyle.

3. Dérivé suivant l'une ou l'autre des revendications 1 et 2, caractérisé en ce que R₂ représente un atome d'hydrogène ou un atome de chlore.

4. Dérivé suivant l'une quelconque des revendications 1 à 3, caractérisé en ce qu'il est constitué par un sel choisi dans le groupe formé par les chlorhydrates, les bromhydrates, les sulfates, les phosphates, les acétates, les citrates, les maléates, les fumarates et les méthanesulfonates.

5. Dérivé suivant l'une quelconque des revendications 1 à 4, caractérisé en ce qu'il est choisi dans le groupe comprenant :
la 11-(4-méthylpipérazin-1-yl)-5H-pyrido(4,3-b)benzo-1,5-diazépine;
la 6-(4-méthylpipérazin-1-yl)pyrido(2,3-b)benzo-1,4-thiazépine;
le fumarate de 8-chloro-6-(4-méthylpipérazin-1-yl)pyrido(2,3-b)benzo-1,4-thiazépine;
le fumarate de 8-chloro-5-(4-méthylpipérazin-1-yl)pyrido(2,3-b)benzo-1,5-oxazépine;
le fumarate de 5-(4-méthylpipérazin-1-yl)-8-méthylpyrido(2,3-b)benzo-1,5-oxazépine;
le fumarate de 6-(4-méthylpipérazin-1-yl)pyrido(2,3-b)benzo-1,4-oxazépine;
le fumarate de 8-chloro-6-(4-méthylpipérazin-1-yl)pyrido(2,3-b)benzo-1,4-oxazépine;
la 6-(4-méthylpipérazin-1-yl)-11H-pyrido(2,3-b)benzo-1,4-diazépine;
la 8-chloro-6-(4-méthylpipérazin-1-yl)-11H-pyrido(2,3-b)benzo-1,4-diazépine;
la 6-(4-méthylpipérazin-1-yl)-8-méthyl-11H-pyrido(2,3-b)benzo-1,4-diazépine;
la 9-chloro-6-(4-méthylpipérazin-1-yl)-11H-pyrido(2,3-b)benzo-1,4-diazépine;
la 8-fluoro-6-(4-méthylpipérazin-1-yl)-11H-pyrido(2,3-b)benzo-1,4-diazépine;
la 5-formyl-11-(4-méthylpipérazin-1-yl)-5H-pyrido(4,3-b)benzo-1,5-diazépine;
la 11-formyl-5-(4-méthylpipérazin-1-yl)-11H-pyrido(2,3-b)benzo-1,5-diazépine;
la 11-trifluorométhylcarbonyl-5-(4-méthylpipérazin-1-yl)-11H-pyrido(2,3-b)benzo-1,5-diazépine;
la 11-formyl-6-(4-méthylpipérazin-1-yl)-11H-pyrido(2,3-b)benzo-1,4-diazépine;
la 5-(4-méthylpipérazin-1-yl)dibenzo(b,f)1,4-sélénazépine;
la 6-(4-méthylpipérazin-1-yl)-11-méthyl-11H-pyrido(2,3-b)benzo-1,4-diazépine.

6. Composition pharmaceutique, caractérisée en ce qu'elle comprend un dérivé suivant l'une quelconque des revendications 1 à 5, y compris le maléate de 5-(4-méthylpipérazin-1-yl)pyrido(2,3-b)benzo-1,5-oxazépine et la 10-(4-méthylpipérazin-1-yl) pyrido[4,3-b][1,4]-benzothiazépine), en mélange avec un excipient pharmaceutiquement acceptable et éventuellement d'autres agents thérapeutiques.

7. Utilisation d'un dérivé suivant l'une quelconque des revendications 1 à 6, pour la production de préparations ayant une activité au niveau du système nerveux central ou périphérique, telle qu'antidépressive, antipsychotique, anxiolytique, neuroleptique ou sédative.

## Claims

1. A methylpiperazinoazepine derivative of general formula (I): 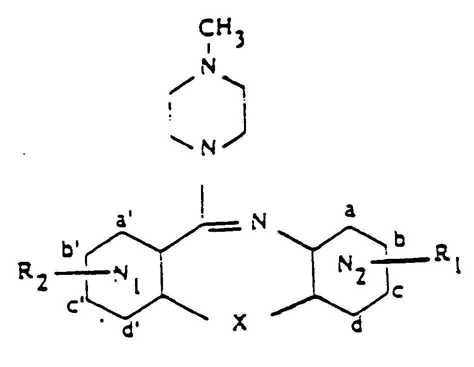 in which:
X is an oxygen, sulphur or selenium atom or a group NH or NR₃, where R₃ is a group 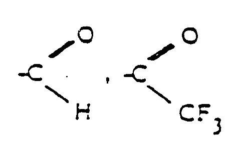 or a branched or unbranched alkyl group having 1 to 4 carbon atoms;
R₁ is a hydrogen atom, a halogen atom or a branched or unbranched alkyl group having 1 to 4 carbon atoms;
R₂ is a hydrogen atom, a halogen atom or a branched or unbranched alkyl group having 1 to 4 carbon atoms; and
N₁ is a benzene ring and N₂ is a pyridine ring or vice-versa, where the nitrogen atom of the pyridine ring is in the b- or b'-position or d- or d'-position, with the proviso that if R₁ and R₂ are hydrogen and X is sulphur, oxygen or a group NH, N₁ is the pyridine ring and N₂ is the benzene ring, it being impossible for the pyridine nitrogen to be in the d'-position, and N₁ and N₂ can also both be benzene rings if X is a selenium atom, and
the derivative 6-(4-methylpiperazin-1-yl)dipyrido(2,3-b:3',2'-f)-1,4-thiazepine, with the exclusion of 10-(4-methylpiperazin-1-yl)pyrido[4,3-b][1,4]benzothiazepine, or a pharmaceutically acceptable salt of these derivatives.

2. A derivative according to Claim 1, characterized in that R₁ is a hydrogen atom, a chlorine atom, a fluorine atom or a methyl group.

3. A derivative according to one or other of Claims 1 and 2, characterized in that R₂ is a hydrogen atom or a chlorine atom.

4. A derivative according to any one of Claims 1 to 3, characterized in that it consists of a salt selected from the group comprising hydrochlorides, hydrobromides, sulphates, phosphates, acetates, citrates, maleates, fumarates and methanesulphonates.

5. A derivative according to any one of Claims 1 to 4, characterized in that it is selected from the group comprising:
11-(4-methylpiperazin-1-yl)-5H-pyrido(4,3-b)benzo-1,5-diazepine;
6-(4-methylpiperazin-1-yl)pyrido(2,3-b)benzo-1,4-thiazepine;
8-chloro-6-(4-methylpiperazin-1-yl)pyrido(2,3-b)benzo-1,4-thiazepine fumarate;
8-chloro-5-(4-methylpiperazin-1-yl)pyrido(2,3-b)benzo-1,5-oxazepine fumarate;
5-(4-methylpiperazin-1-yl)-8-methylpyrido(2,3-b)benzo-1,5-oxazepine fumarate;
6-(4-methylpiperazin-1-yl)pyrido(2,3-b)benzo-1,4-oxazepine fumarate;
8-chloro-6-(4-methylpiperazin-1-yl)pyrido(2,3-b)benzo-1,4-oxazepine fumarate;
6-(4-methylpiperazin-1-yl)-11H-pyrido(2,3-b)benzo-1,4-diazepine;
8-chloro-6-(4-methylpiperazin-1-yl)-11H-pyrido(2,3-b)benzo-1,4-diazepine;
6-(4-methylpiperazin-1-yl)-8-methyl-11H-pyrido(2,3-b)benzo-1,4-diazepine;
9-chloro-6-(4-methylpiperazin-1-yl)-11H-pyrido(2,3-b)benzo-1,4-diazepine;
8-fluoro-6-(4-methylpiperazin-1-yl)-11H-pyrido(2,3-b)benzo-1,4-diazepine;
5-formyl-11-(4-methylpiperazin-1-yl)-5H-pyrido(4,3-b)benzo-1,5-diazepine;
11-formyl-5-(4-methylpiperazin-1-yl)-11H-pyrido(2,3-b)-benzo-1,5-diazepine;
11-trifluoromethylcarbonyl-5-(4-methylpiperazin-1-yl)-11H-pyrido(2,3-b)benzo-1,5-diazepine;
11-formyl-6-(4-methylpiperazin-1-yl)-11H-pyrido(2,3-b)-benzo-1,4-diazepine;
5-(4-methylpiperazin-1-yl)dibenzo(b,f)-1,4-selenazepine; and
6-(4-methylpiperazin-1-yl)-11-methyl-11H-pyrido(2,3-b)-benzo-1,4-diazepine.

6. A pharmaceutical composition, characterized in that it comprises a derivative according to any one of Claims 1 to 5, including 5-(4-methylpiperazin-1-yl)-pyrido(2,3-b)benzo-1,5-oxazepine maleate and 10-(4-methylpiperazin-1-yl)pyrido[4,3-b][1,4]benzothiazepine, mixed with a pharmaceutically acceptable excipient and optionally with other therapeutic agents.

7. Use of a derivative according to any one of Claims 1 to 6 for the production of preparations which are active on the central or peripheral nervous system, for example as antidepressants, antipsychotics, anxiolytics, neuroleptics or sedatives.

## Patentansprüche

1. Methylpiperazinoazepin-Derivat, entsprechend der allgemeinen Formel (I): 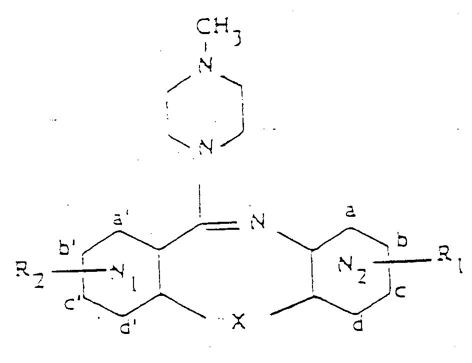 wobei:
X ein Sauerstoffatom, ein Schwefelatom oder ein Selenatom oder eine Gruppe NH oder NR₃ darstellt, wobei R₃ eine Gruppe 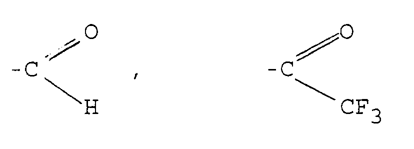 oder Alkyl mit 1 bis 4 Kohlenstoffatomen, verzweigt oder nicht, darstellt;
R₁ ein Wasserstoffatom, ein Halogenatom oder eine Alkylgruppe mit 1 bis 4 Kohlenstoffatomen, verzweigt oder nicht, darstellt;
R₂ ein Wasserstoffatom, ein Halogenatom oder eine Alkylgruppe mit 1 bis 4 Kohlenstoffatomen, verzweigt oder nicht, darstellt; und
N₁ einen Benzolring darstellt und N₂ einen Pyridinring darstellt oder umgekehrt, wobei das Stickstoffatom des Pyridinrings in Position b, b', d oder d' angeordnet ist, unter der Bedingung daß, falls R₁ und R₂ Wasserstoff darstellen und X Schwefel, Sauerstoff oder eine Gruppe NH darstellt, N₁ Pyridin ist und N₂ Benzol ist, wobei der Pyridin-Stickstoff nicht in Position d' sein kann und wobei N₁ und N₂ darüber hinaus beide Benzol sein können, falls X ein Selenatom darstellt, und
das Derivat 6-(4-Methylpiperazin-1-yl)dipyrido-(2,3-b:3',2'-f)1,4-thiazepin, mit Ausnahme von 10-(4-Methylpiperazin-1-yl)pyrido[4,3-b][1,4]benzothiazepin, oder ein pharmazeutisch verträgliches Salz dieser Derivate.

2. Derivat entsprechend Anspruch 1, dadurch charakterisiert, daß R₁ ein Wasserstoffatom, ein Chloratom, ein Fluoratom oder eine Methylgruppe darstellt.

3. Derivat entsprechend einem oder dem anderen der Ansprüche 1 oder 2, dadurch charakterisiert, daß R₂ ein Wasserstoffatom oder ein Chloratom darstellt.

4. Derivat entsprechend einem der Ansprüche 1 bis 3, dadurch charakterisiert, daß es ein Salz ist, ausgewählt aus der aus Hydrochloriden, Hydrobromiden, Sulfaten, Phosphaten, Acetaten, Citraten, Maleaten, Fumaraten und Methansulfonaten gebildeten Gruppe.

5. Derivat entsprechend einem der Ansprüche 1 bis 4, dadurch charakterisiert, daß es ausgewählt ist aus der Gruppe umfassend:
11-(4-Methylpiperazin-1-yl)-5H-pyrido(4,3-b)benzo-1,5-diazepin;
6-(4-Methylpiperazin-1-yl)pyrido(2,3-b)benzo-1,4-thiazepin;
Fumarat von 8-Chloro-6-(4-methylpiperazin-1-yl)pyrido-(2,3-b)benzo-1,4-thiazepin;
Fumarat von 8-Chloro-5-(4-methylpiperazin-1-yl)pyirdo-(2,3-b)benzo-1,5-oxazepin;
Fumarat von 5-(4-Methylpiperazin-1-yl)-8-methylpyrido-(2,3-b)benzo-1,5-oxazepin;
Fumarat von 6-(4-Methylpiperazin-1-yl)pyrido(2,3-b)benzo-1,4-oxazepin;
Fumarat von 8-Chloro-6-(4-methylpiperazin-1-yl)pyrido-(2,3-b)benzo-1,4-oxazepin;
6-(4-Methylpiperazin-1-yl)-11H-pyrido(2,3-b)benzo-1,4-diazepin;
8-Chloro-6-(4-methylpiperazin-1-yl)-11H-pyrido(2,3-b)-benzo-1,4-diazepin;
6-(4-Methylpiperazin-1-yl)-8-methyl-11H-pyrido(2,3-b)-benzo-1,4-diazepin;
9-Chloro-6-(4-methylpiperazin-1-yl)-11H-pyrido(2,3-b)-benzo-1,4-diazepin;
8-Fluoro-6-(4-methylpiperazin-1-yl)-11H-pyrido(2,3-b)-benzo-1,4-diazepin;
5-Formyl-11-(4-methylpiperazin-1-yl)-5H-pyrido(4,3-b)-benzo-1,5-diazepin;
11-Formyl-5-(4-methylpiperazin-1-yl)-11H-pyrido(2,3-b)-benzo-1,5-diazepin;
11-Trifluormethylcarbonyl-5-(4-methylpiperazin-1-yl)-11H-pyrido(2,3-b)benzo-1,5-diazepin;
11-Formyl-6-(4-methylpiperazin-1-yl)-11H-pyrido(2,3-b)-benzo-1,4-diazepin;
5-(4-Methylpiperazin-1-yl)dibenzo(b,f)1,4-selenazepin;
6-(4-Methylpiperazin-1-yl)-11-methyl-11H-pyrido(2,3-b)-benzo-1,4-diazepin.

6. Pharmazeutische Zusammensetzung, dadurch charakterisiert, daß sie ein Derivat entsprechend einem der Ansprüche 1 bis 5 enthält, darin eingeschlossen das Maleat von 5-(4-Methylpiperazin-1-yl)pyrido(2,3-b)benzo-1,5-oxazepin und 10-(4-Methylpiperazin-1-yl)pyrido-[4,3-b][1,4]benzothiazepin), im Gemisch mit einem pharmazeutisch verträglichen Träger und gegebenenfalls anderen therapeutischen Mitteln.

7. Verwendung eines der Derivate entsprechend einem der Ansprüche 1 bis 6 für die Herstellung von Präparaten mit einer Aktivität für das zentrale oder periphere Nervensystem wie antidepressive, antipsychotische, anxiolytische, neuroleptische oder sedative Aktivität.
